# EUROPEAN PATENT APPLICATION

(11) **EP 2 366 708 A1**
(43) Date of publication of application: **21.09.2011**
(21) Application number: 10157102.4
(22) Date of filing: 19.03.2010
(51) Int. Cl.: C07K 14/005, C07K 14/08, C12N 7/00, G01N 33/569, C12N 15/40, C07K 16/10, C12Q 1/70, A61K 39/12

(54) **Canine astrovirus AstV strain Bari/08 identified in dogs with gastro-enteritis**

(71) Applicant: Martella, Vito, 70010 Casamassima (BA) (IT)
(72) Inventor: Martella, Vito, 70010 Casamassima (BA) (IT); Buonavoglia, Canio, 70016 Noicattaro (BA) (IT); Decaro, Nicola, 70126 Torre a Mare (BA) (IT); Elia, Gabriella, 70100 Bari (IT)
(74) Representative: Banfi, Paolo

(57) **Abstract**

The invention provides an isolated canine virus (called "strain Bari/08") which was found to belong to the *Astroviridae* family and was identified in dogs with gastro-enteritis. The invention further provides polynucleotides and proteins isolated therefrom, methods and assays using same and vaccine preparations containing the virus or antigens thereof.

## Description

The present invention provides a virus isolated from dogs and associated with gastro-enteric diseases. The virus was characterized molecularly and found to belong to the *Astroviridae* family. Specific antibodies against the virus were identified in convalescent dogs and the presence of the virus was correlated with enteritis in the screened animals. The invention further provides polynucleotides and proteins isolated from the virus, methods and assays using virus polynucleotide sequences, protein antigens and/or antibodies thereto, diagnostic compositions and kits for the identification of infectious state caused by the virus and vaccine preparations containing the virus or antigens thereof.

### Background of the invention

The family *Astroviridae* includes human and animal small-rounded viruses (SRVs) with a peculiar star-like surface when observed by electron microscopy (EM). Astroviruses (AstVs) are non enveloped and their genome is composed of a plus-sense single-stranded RNA of 6.4-7.3 in size, containing 3 ORFs and with a 3' poly-A tail (*Méndez and Arias, 2007*). Two ORFs, located at the 5' end of the genome (ORF1a and ORF1b), encode non structural proteins, while ORF2, located at the 3' end, encodes the capsid protein *(Méndez and Arias, 2007).* AstVs were first identified by EM in 1975 in Scotland in stools of infants hospitalized with diarrhea *(Madeley and Cosgrove, 1975).* Subsequently, similar SRVs were identified from several mammalian and avian species *(Snodgrass and Gray, 1977; Woode and Bridger, 1978; Tzipori et al., 1981; Bridger 1980; Hoshino et al., 1984; Kjeldsberg and Hem, 1985; Englund et al, 2002; McNulty et al, 1980; Todd et al., 2009; Imada et al., 2000)* including bats *(Chu et al., 2008),* and aquatic mammals *(Rivera et al., 2009).* AstV infection is associated with gastro-enteritis in most animal species and humans AstVs are regarded as the second or third most common cause of viral diarrhoea in children *(Méndez and Arias, 2007).* Avian AstVs have also been associated with extra-intestinal diseases, such as nephritis in chicken *(Imada et al., 2000)* and hepatitis in ducks *(Todd et al., 2009).*

SRVs have been detected only occasionally in dogs by EM. In some cases, due to their morphological similarities (about 25-35 nm in size, rounded shape, absence of envelope), this definition has been used to refer to either AstV- calicivirus- or picornavirus-like particles. AstV-like particles were first detected in beagle pups with diarrhoeal disease in USA, 1980, in mixed infection with canine parvovirus type-2 (CPV2) and canine coronaviruses (CCoV) *(Williams, 1980).* AstV-like particles were also detected in 3/157 normal faeces (but not in 29 diarrheal samples) in a survey in Australia, 1984 *(Marshall, 1984).* In a large survey in Germany, SRVs were identified in 41/4044 (<1%) faces of diarrhoeal dogs *(Vieler and Herbst, 1995).* More recently, AstVs have been identified in dogs with enteric signs and characterised molecularly, suggesting that the detected viruses may represent a distinct AstV species *(Toffan et al., 2009).*

### Description of the invention

The invention provides an isolated canine AstV (called "strain Bari/08") which was identified in dogs with gastro-enteritis that tested negative for all other canine pathogens.

Upon sequence analysis, the virus was characterised as a member of the *Mamastrovirus* genus, and displayed the highest identity in the polymerase complex (in a short 110-aa overlap) (100% aa) and in the full-length capsid protein (83.5% aa) to the canine strain Italy05 *(Toffan et al., 2009).* However, the genetic diversity between strain Italy05 and Bari/08 appeared markedly higher than that observed between human AstVs of a same serotype. Specifically, between strain Italy05 and Bari/08, a high degree of variation occurred between aa 422 and 668 (50% aa identity), whilst high conservation was found between aa 1 and 422 (98,3%) and in the C-terminal residues downstream aa 668 (95,7%). The hypervariable C-terminal region downstream aa 415 is believed to form the spikes of the virion, and to interact with the cell receptors *(Krishna, 2005)* as neutralizing monoclonal antibodies have been mapped to this variable domain *(Bass and Upadhyayula, 1997; Sanchez-Fauquier et al., 1994).*

Thus, the isolated canine virus displays distinctive genetic traits compared to known astrovirus strains. In addition, unlike previously identified canine astroviruses, strain Bari/08 was successfully adapted to grow on a canine cell line (MDCK) after three serial passages and viral replication induced a clear cytophatic effect. Also, viral antigens were visualized in the cytoplasm of infected cells by IF.

The isolated virus according to the invention:
i) encodes a polypeptide SEQ ID NO: 1 at the carboxy-terminus of the polymerase complex and a capsid protein SEQ ID NO:2; or a polypeptide having at least 95%, preferably at least 97% sequence identity to SEQ ID NO:1 or SEQ ID NO:2 over the entire sequence;
ii) is able to grow on MDCK cells;
iii) causes gastro-enteritis in dogs.

In one embodiment, the isolated virus contains a polynucleotide encoding a polypeptide fragment at the carboxy terminus of the polymerase complex, wherein said polynucleotide is either SEQ ID NO:3 or a polynucleotide hybridizing to full-length SEQ ID NO:3 under stringent conditions.

In a further embodiment, the isolated virus contains a polynucleotide encoding the capsid protein, wherein said polynucleotide is either SEQ ID NO:4 or a polynucleotide hybridizing to full-length SEQ ID NO:4 under stringent conditions.

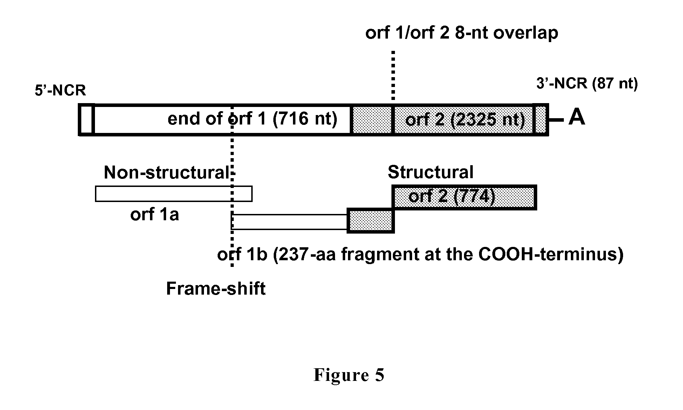

In a further embodiment, the isolated virus contains two open reading frames (ORF), respectively designated ORF1b and ORF2, that encode for the polypeptides SEQ ID NO: 1 and SEQ ID NO:2 and overlap each other over a 8-nucleotide region between the termination codon of ORF1b and the initiation codon of ORF2 (Figure 5).

In a preferred embodiment, the isolated virus contains a 3143 nt-long sequence (SEQ ID NO:5) spanning the 3' end of ORF1b (SEQ ID NO:3), the full-length ORF2 (SEQ ID NO:4) and the 3' non coding region to the poly-A tail.

In a further embodiment, the invention provides either an isolated canine astrovirus polypeptide selected from SEQ ID NO: 1 and SEQ ID NO:2, or a canine astrovirus polypeptide having at least 95%, preferably at least 97% sequence identity to full-length SEQ ID NO:1 or SEQ ID NO:2. These virus-specific proteins or antigenic fragments thereof can be used in the development of diagnostic tools and assays, as described below.

In a further embodiment, the invention provides either an isolated canine astrovirus polynucleotide selected from SEQ ID NO:3, SEQ ID NO:4 and SEQ ID NO:5 or an isolated canine astrovirus polynucleotide hybridizing to the full-length sequence SEQ ID NO:3, SEQ ID NO:4 or SEQ ID NO:5 under stringent conditions.

As used herein, the term "stringent conditions" indicates that the hybridization occurs at a temperature 5-10 °C below the Tm and at 0.15 M NaCl ionic strenght.

A further embodiment of the invention relates to an antibody specifically binding to the virus or to an isolated antigen thereof. By using the isolate Bari/08 as antigen, specific antibodies were identified in the convalescent dog and in 59.0% of the tested canine sera. AstV-specific antibodies were detected in dogs with more than 3 months of age. According to the invention, the antibodies can be monoclonal, polyclonal or single chain antibodies or antibody fragments such as Fab, F(ab)2, Fv or scFV fragments.

In a further embodiment, the invention provides a method for determining the presence of the astrovirus in a dog, comprising the steps of: (i) providing a dog cell, tissue or body fluid sample, (ii) contacting that sample with an antibody that binds the canine astrovirus herein disclosed and (iii) detecting the binding of the antibodies to the sample, whereby detection of a binding is indicative of the presence of the virus in the tested sample. Alternatively, the dog sample can be tested for the presence of antibodies by contacting it with a virus antigen adhered to a solid support and determining the formation of an antigen-antibody complex, whereby detection of the antigen-antibody complex is indicative of virus presence in the sample.

Virus-specific proteins, preferably the capside protein SEQ ID NO:2 or an antigenic peptide thereof can be used to assemble immuno or radio-immuno assays, such as ELISA or Western blot, or for the development of antibodies.

In a yet further embodiment, the invention provides a method for virus detection in dogs which comprises (i) providing a sample of dog cells, tissues or body fluid and (ii) amplifying a virus specific sequence selected from SEQ ID NO:3 and SEQ ID NO:4 or a fragment thereof by means of PCR techniques, whereby the detection of a sequence amplicon is indicative of virus presence in the tested sample. Suitable forward and reverse primers may be selected on the basis of their ability to hybridize to SEQ ID NO:3 and SEQ ID NO:4 under stringent conditions, as above described, and allow the elongation of respective strands upon polymerase addition. In a preferred embodiment, the oligonucleotide primers are selected from SEQ ID NO:6 and SEQ ID NO:7.

In a yet further embodiment, the invention provides a diagnostic kit that contains reagents, solutions, buffers, antibodies and/or enzymes in separate compartments, and a leaflet with user instructions to carry out a biological or immunoenzimatic assay or a PCR reaction as above described.

A further embodiment of the invention relates to a vaccine composition for immunizing dogs against astrovirus infection. The vaccine contains an isolated or recombinant virus immunogenic protein, which is preferably selected from SEQ ID NO:2, or an avirulent antigenic product obtained by attenuation or inactivation of the astrovirus pathogenic strain herein disclosed. The virus can be isolated from the stools of infected animals, propagated in animal cells, recovered and concentrated to an appropriate viral titer, inactivated by chemical treatment or attenuated by repeated passages in suitable cells, especially in canine cells. Attenuation of the astrovirus isolate according to the invention can be performed with known methods (see for example Gouvea et al., Virology 126:240-247, 1983). The astrovirus strain can be easily identified by means of specific molecular markers, such as the polymerase complex or capsid proteins disclosed herein.

The vaccine composition according to the invention may contain carriers, stabilisers, preservatives, buffers, diluents and adjuvants commonly used in vaccine technology, such as aluminium hydroxide, water/oil emulsions and the like. The vaccine can be administered by several routes, preferably by the parenteral, intramuscular or subcutaneous routes.

The vaccine composition according to the invention is advantageously used to prevent or treat viral gastro-enteritis in dogs, particularly in young animals and pups.

### Description of the Figures

**Figure 1**: Cytopathic effect induced by AstV replication in 24-hours infected MDCK cells at the 3^{rd} passage.
**Figure 2****:** Indirect IF on MDCK cells infected by canine AstV.
**Figure 3****:** EM observation on the pooled faecal samples.
**Figure 4****:** Phylogenetic tree based on the full-length amino acid sequence of the capsid protein of AstVs of avian and mammalian origin. The tree was elaborated with the neighbour-joining method without any distance correction. The accession numbers of the AstV strains used are listed in Table 1.
**Figure 5****:** AstV genome organization. The portion of the genome of the canine AstV Ba/08 (from the 3' end of ORF1b to the poly A tail) 3.1Kb in length, is shown in grey.

### Experimental section

**History of the animal and virological examinations.** A 90-day-old mixed-breed pup developed a gastro-enteric disease with watery diarrhoea. The pup was hospitalised 2 days after the onset of the gastro-enteric disease due to severe dehydration. After 2 days of illness, the dog recovered completely. At the time of hospitalization, the faeces were collected and screened for the presence of common canine viral pathogens by either gel-based PCR or quantitative PCR and RT-PCR. DNA and RNA extracts were prepared using the DNAeasy® and QIAamp® viral RNA kit (Qiagen GmbH, Hilden, Germany). The faeces of the pup tested negative to all known viral pathogens, including (canine parvovirus type-2 (CPV-2), canine coronaviruses (CCoV), distemper virus (CDV), adenoviruses (CAV-1 and CAV-2), reovirus, rotavirus and calicivirus. Unexpectedly, the stools tested positive to AstV using a broadly reactive primer pair, targeted to the ORB1b region *(Chu et al., 2008).* After the detection of astroviral RNA in the faeces of the dog, the animal was kept under observation. Faecal samples were collected daily from days post-hospitalization (dph) 2 to 30 in order to monitor virus shedding. Astroviral RNA was detected until dph 10. In addition, serum samples were obtained from the dog at hospitalization and at dph 14 and 28.

**Cultivation on canine cell lines.** Faecal samples collected from dph 2 to 6 were pooled and then used in attempts to adapt the virus to growth in tissue cultured cells. Evidence was obtained for viral growth in canine cell lines (MDCK) grown in Dulbecco's modified Eagle' medium (DMEM) supplemented with trypsin 20 µg/ml (type IX, Sigma), after the 3^{rd} serial passage. The cytopathic effect was characterized by enlargement and/or detaching of cells and by appearance of fine granules in the cytoplasm. Astroviral antigen in the infected cells was revealed by indirect immunofluorescence (IF) using the convalescent serum (dph 28) of the dog and a fluoresceine-coniugated anti-dog serum (Sigma-Aldrich Srl, Milan, Italy).

**Electron microscopy observation.** Faecal samples collected from dph 2 to 6 were pooled and processed for EM observation. Briefly, the faeces were diluted 1:10 in distilled water, vortexed and centrifuged for 20 min at 4000×g and again for 10 min at 9300×g for clarification. The supernatant was then ultracentrifuged (Beckman Airfuge) for 15 min at 82000×g. After negative staining with 2% sodium phosphotungstate (pH 6.8), samples were examined using a Philips CM10 electron microscope.

**Sequence and phylogenetic analysis of the astrovirus strain.** The amplicons obtained with the ORF1b-astrovirus-specific primers *(Chu et al., 2008)* were excised from the gel and purified using a QIAquick gel extraction kit (Qiagen GmbH, Hilden, Germany). The fragment was then subjected to direct sequencing using BigDye Terminator Cycle chemistry and 3730 DNA Analyzer (Applied Biosystems, Foster, CA). Basic Local Alignment Search Tool (BLAST; http://www.ncbi.nlm.nih.gov) and FASTA (http://www.ebi.ac.uk/fasta33) with default values were used to find homologous hits.

In order to determine the sequence and genomic organization of the AstV strain, a 3.2 kb region at the 3' end of the genome was amplified by RT-PCR as described by Wang et al. *(2005).* cDNA was synthesized by Superscript III First-Strand cDNA synthesis kit (Invitrogen Ltd, Paisley, UK) with primer VN₃T₂₀ (5'-GAGTGACCGCGGCCGCT₂₀-3'). PCR was then performed with TaKaRa La Taq polymerase (Takara Bio Europe S.A.S.

Saint-Germain-en-Laye, France) with forward primer and VN₃T₂₀. Finally, the amplicon was purified and cloned by using TOPO^{®} XL Cloning Kit (Invitrogen Ltd, Paisley, UK). Additional primers also were designed to determine the complete 3.2 kb sequence by an overlapping strategy.

Sequence editing and multiple alignments were performed with Bioedit software package vers. 2.1 *(Hall et al., 1999).* Phylogenetic analysis (Neighbor-Joining and UPGMA) with bootstrap analysis (1,000 replicates) and no distance correction was conducted by using the MEGA software package v4.0 *(Tamura et al., 2007).*

**Analysis of the capsid protein of strain Bari/08/ITA.** Pair-wise identity in the full-length capsid protein of strain Ca/Bari/08/ITA to a selection of AstV strains was determined using multiple alignments generated with Bioedit software package vers. 2.1 *(Hall et al., 1999)* The values were calculated by the uncorrected distance method using a 31-sequence alignment without removing the gaps, including sequences of human and animal AstVs. The strain and sequences used are listed in table 1.

**Table 1: list of AstV strains (with the Accession numbers) included in the present study. Only strains for whom complete sequence of the ORF2 (encoding for the capsid protein) was available were included in the study. Strain designation of the viruses is reported as in the original studies and/or as found in the GenBank databases.**

| **Species of origin** | **Type** | **strain** | **Accession number** |
|---|---|---|---|
| | | | |
| Human | Type 1 | Dresden | AY720892 |
| Human | Type 2 | Oxford | L06802 |
| Human | Type 3 | WH1859 | DQ630763 |
| Human | Type 4 | Goiania/GO/12/95/Brazil | DQ070852 |
| Human | Type 5 | Goiania/GO/12/94/Brazil | DQ028633 |
| Human | Type 6 | 192-BJ07-CHN | GQ495608 |
| Human | Type 7 | Oxford | AF248738 |
| Human | Type 8 | Yuc-8 | AF260508 |
| Human | VA1 | VA1 | FJ973620 |
| Human | VA2 | VA2/human/Stl/WD0680/2009 | GQ502193 |
| Human | MLB1 | MLB1 | FJ222451 |
| Human | HMO A | NI-295 | NC_013443 |
| Human | HMO B | NI-196 | GQ415661 |
| Human | HMO C | NI-3010 | GQ415662 |
| Ovine | | | Y15937 |
| Mink | | | AY 179509 |
| Porcine | | Tokushima83-74 | AB037272 |
| Feline | | Bristol | AF056197 |
| Dog | | Italy/2005 | FM213332 |
| California sea lion | Type 1 | CSL1 | FJ890351 |
| California sea lion | Type 2 | CSL2 | FJ890352 |
| Bottlenose dolphin | Type 1 | Bdl | FJ890355 |
| Bat | Type 1 | AFCD337 | EU847155 |
| Bat | LC03 | Hp/Guangxi/LC03/2007 | FJ571074 |
| Bat | LD38 | Tm/Guangxi/LD38/2007 | FJ571065 |
| Duck | | C-NGB | NC_012437 |
| Turkey | Type 1 | | Y15936 |
| Turkey | Type 2 | | NC_005790 |
| Turkey | Type 3 | 2001 | AY769616 |
| Chicken | ANV-1 | G-4260 | AB033998 |

**Collection of faecal samples and extraction of DNA and RNA.** A total of 110 stool samples were screened. The samples were a subset of a collection obtained between January and December 2007 from young dogs (aged 1 to 6 months) of various origin (animals housed in shelters and/or pet shops and hospitalized animals) with signs of mild to severe gastro-enteritis. DNA and RNA extracts were prepared using the DNAeasy® and QIAamp® viral RNA kit (Qiagen GmbH, Hilden, Germany).

**Screening for canine enteric and non-enteric viral pathogens**. All faecal samples were screened for the presence of common canine viral pathogens by either gel-based PCR or quantitative PCR and RT-PCR (qPCR and qRT-PCR). The samples were screened for CPV-2 *(Decaro et al., 2005),* enteric CCoVs *(Decaro et al., 2004),* and CDV *(Elia et al., 2004)* by qPCR or qRT-PCR, using a LightCycler instrument (i-Cycler iQTM Real-Time Detection, Bio-Rad Laboratories Srl) and IQ^{™} Supermix (Bio-Rad Laboratories Srl), after reverse transcription of RNA with MuLV reverse transcriptase (Applied Biosystem, Monza, Italy) for RNA viruses. Screening for CAV-1 and CAV-2 *(Hu et al., 2001),* respiratory coronavirus (CrCoV) *(Decaro et al. 2008),* rotavirus *(Gentsch et al., 1992)* and norovirus *(Vennema and Koopmans, 2002)* was accomplished by conventional PCR or RT-PCR, in a Gene Amp^{®} PCR System 9700 (Applied Biosystem, Monza, Italy), using TaKaRa La Taq polymerase (Takara Bio Europe S.A.S. Saint-Germain-en-Laye, France) for DNA viruses and Superscript III One step RT-PCR kit (Invitrogen Ltd, Paisley, UK) for RNA viruses.

**Screening for astroviruses by RT-PCR.** The RNA extracts were screened using a primer pair specific for canine AstVs. Primers 625F-1 (GTA CTA TAC CRT CTG ATT TAA TT) and 626R-1 (AGA CCA ARG TGT CATAGT TCA G) were designed on the ORF1b sequence to amplify selectively a fragment of 300 bp in length.

**Serological investigations**. Detection of specific antibodies for strain Bari/08 in the canine serum samples was performed using an indirect immunofluorescence (IF) assay. A total of 54 serum samples, obtained from dogs aged 2 months to 7 years, were screened. The virus Bari/08 was used as antigen. Confluent MDCK cell monolayers were infected and gently harvested in the presence of advanced cytopathic effect (affecting at least 70-80% of the cells). Infected cells were distributed onto multispot glass slides and dried at 37°C. The slides were fixed in 100% cold acetone at room temperature for 30 min, and stored at -80°C until used. Ten microliters of each serum sample, at a dilution 1:50, were layered onto the cells and incubated at 37°C for 30 min. After three washes with phosphate buffered solution, antiserum to dog IgG conjugated with fluorescein isothiocyanate (Sigma-Aldrich srl, Milan, Italy) was added and the slides were incubated at 37°C for 30 min. After three washes with PBS, the slides were observed under a fluorescent microscope and the antibody titres were calculated as the highest serum dilutions still giving cytoplasmatic fluorescence in the antigen preparations.

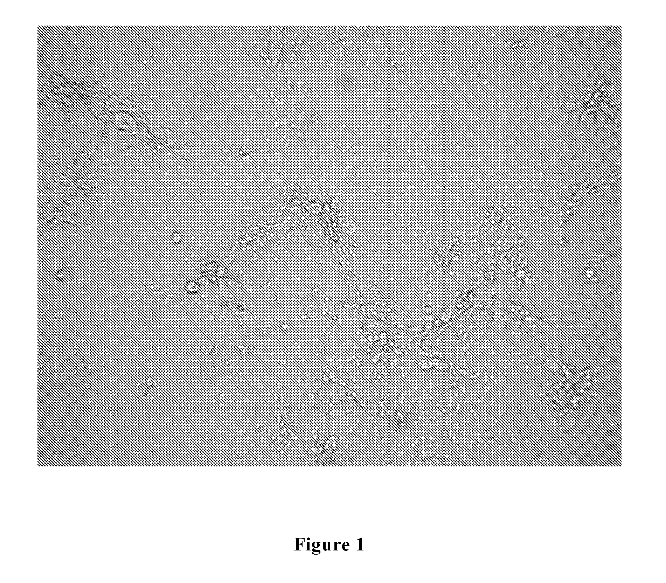

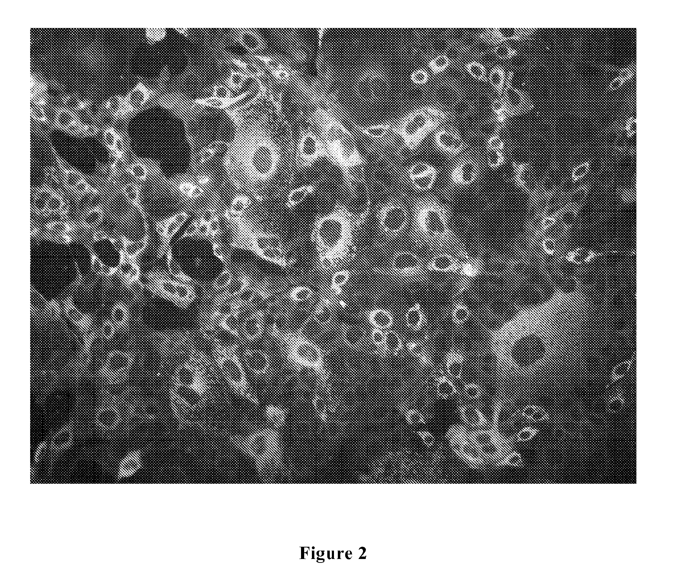

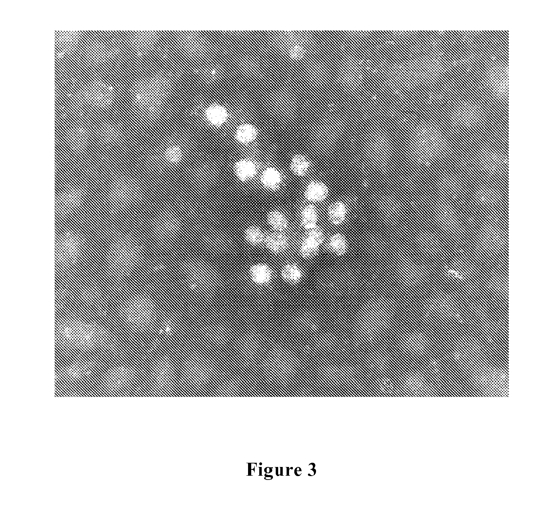

**Characterization of the virus isolate as AstV**. The canine AstV Bari/08 was adapted to grow on MDCK cells. After the 3^{rd} serial passage, a clear cytopathic effect was observed, characterized by enlargement and/or detaching of cells and by appearance of fine granules in the cytoplasm (Figure 1). Viral antigens were observed in the cells as fine granules dispersed in the cytoplasm, and aggregating in perinuclear position (Figure 2). Also, SRVs were identified in pooled faecal samples by EM observation (Figure 3).

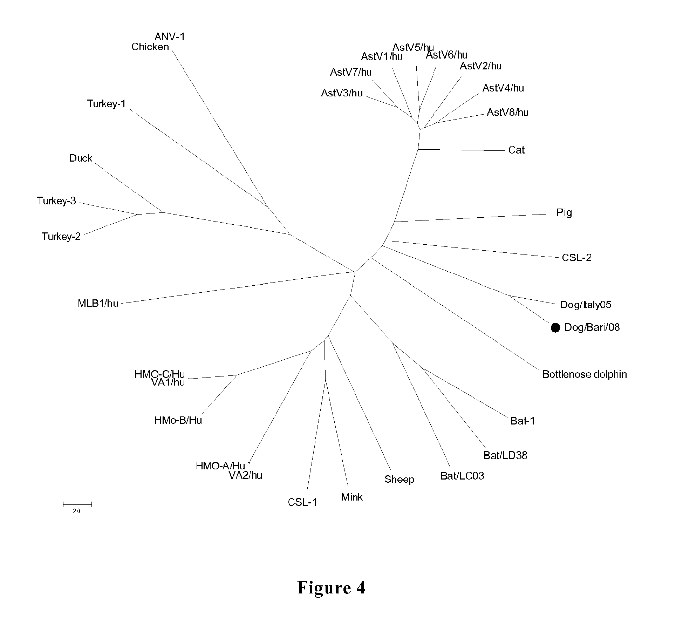

**Sequence and phylogenetic analysis of the isolate Bari/08**. A 3143 nt-long sequence was determined, spanning the 3' end of ORF1b, the full-length ORF2 and the 3' non coding region (NCR) to the poly-A tail. The 3' end of ORF1b comprised 716 nt, encoding for a 237-aa polypeptide fragment at the COOH-terminus of the polymerase complex. In this 237-aa fragment, the highest identity was 79.6% to California Sea Lion type-2 AstV. However, identity was 100% to shorter fragments (111 aa) of AstV strains detected from dogs *(Toffan et al., 2009).* There was a 8 nt-overlap between the termination codon of ORF1b and the initiation codon of ORF2. The highly conserved nt stretch upstream of ORF2, ATTTGGAGNGGNGGACCNAAN₅-₈ATGNC, believed to be part of a promoter region for synthesis of subgenomic RNA (sgRNA) *(Walter et al., 2001),* was nearly completely conserved in the sequence of strain Bari/08. The ORF2 was 2325 nt in length and encoded for a capsid protein of 774 aa, with a predicted molecular mass of 84.7 kd. The non coding region (NCR) was 87 nt in length. By pair-wise comparison, the highest identity (83.5%) in the capsid protein was found to a canine AstV, strain Dog/Italy05. Identity to other mammalian AstVs ranged from 20.1 to 35.6 % aa, whilst identity to avian AstVs was 13.7-17.3% aa (Table 1). The 6-aa long C-terminus of the VP1 (SRGHAE) was highly conserved with several mammalian AstVs. This motif is within a highly conserved nt stretch, s2m, overlapping the termination codon of ORF2, and shared also by some coronaviruses and picornaviruses *(Jonassen et al., 1998).* By phylogenetic analysis, the strain was clustered with the canine strain Italy05, intermingled with AstVs detected from aquatic mammals in the *Mamastrovirus* genogroup (Figure 4).

**Table 2. Amino acid identity in the capsid protein between the canine viruses Italy/05 and Bari/08 and avian and mammalian AstVs, representative of the various AstV species/types. The values were calculated based on the full-length capsid protein alignment, without removing the gaps.**

| **AstV** | **Italy05** | **Bari/08** |
|---|---|---|
| Human type 1 | 34,1 | 33,6 |
| Human type 2 | 33,2 | 34,0 |
| Human type 3 | 33,9 | 33,9 |
| Human type 4 | 34,7 | 34,4 |
| Human type 5 | 34,2 | 34,8 |
| Human type 6 | 34,9 | 35,2 |
| Human type 7 | 34,3 | 34,4 |
| Human type 8 | 34,5 | 35,0 |
| Human VA1 | 21,9 | 22,0 |
| Human VA2 | 20,3 | 20,7 |
| Human MLB 1 | 22,2 | 21,9 |
| Mink | 22,7 | 23,0 |
| Pig | 35,2 | 35,0 |
| CSL type 1 | 20,3 | 20,1 |
| CSL type2 | 35,1 | 35,6 |
| Bottlenose dolphin | 29,8 | 29,9 |
| Bat type 1 | 20,7 | 20,3 |
| Bat LC03 | 20,4 | 20,6 |
| Bat LD38 | 20,4 | 20,6 |
| Duck | 16,9 | 16,3 |
| Turkey type 1 | 15,1 | 15,2 |
| Turkey type 2 | 16,7 | 17,1 |
| Turkey type 3 | 17,3 | 17,3 |
| Avian nephritis virus-1 | 13,6 | 13,7 |
| Dog Italy/05 | ID | 83,5 |
| Dog Bari/08 | 83,5 | ID |

**Screening for canine enteric and non-enteric viral pathogens and for AstV**. A total of 110 stool samples were screened for common canine viral pathogens and for AstV. By RT-PCR, AstV RNA was detected in 27/110 samples (24,5%), either alone (10/110, 9.0%) or in mixed infections with CPV-2 (5/110, 4.5%) and CCoVs (8/110, 7.3%) or CPV-2+CCoVs (4/110, 3.6%). Sequence analysis of the amplicons confirmed the specific nature of the bands.

**Detection by IF of antibodies specific for strain Bari/08 in dog sera.** Antibodies specific for virus Bari/08 were detected in 32/54 (59.0%) serum samples. The majority (14/22, 63.6%) of the serum samples testing negative were from pups aged less than 3 months, while only 2/32 (6.25%) of the positive sera were from dogs aged less than 3 months (P<0,0001).
1. Bass D. M., and U. Upadhyayula. 1997. Characterization of human serotype 1 astrovirus-neutralizing epitopes. J. Virol. 71(11):8666-8671.
2. Bridger J. C. 1980. Detection by electron microscopy of caliciviruses, astroviruses and rotavirus-like particles in the faeces of piglets with diarrhoea. Vet Rec. 107(23):532-533.
3. Chu D. K., L. L. Poon, Y. Guan, and J.S. Peiris. 2008. Novel astroviruses in insectivorous bats. J. Virol. 82(18):9107-9114.
4. Decaro, N., A. Pratelli, M. Campolo, G. Elia, V. Martella, M. Tempesta, and C. Buonavoglia. 2004. Quantitation of canine coronavirus RNA in the faeces of dogs by TaqMan RT-PCR. J. Virol. Methods 119:145-150.
5. Decaro, N., G. Elia, M. Campolo, C. Desario, V. Mari, A. Radogna, M. L. Colaianni, F. Cirone, M. Tempesta, and C. Buonavoglia. 2008. Detection of bovine coronavirus using a TaqMan-based real-time RT-PCR assay. J. Virol. Methods 151:167-171.
6. Decaro, N., G. Elia, V. Martella, C. Desario, M. Campolo, L. D. Trani, E. Tarsitano, M. Tempesta, and C. Buonavoglia. 2005. A real-time PCR assay for rapid detection and quantitation of canine parvovirus type 2 in the feces of dogs. Vet. Microbiol. 105:19-28.
7. Elia, G., N. Decaro, V. Martella, F. Cirone, M. S. Lucente, E. Lorusso, T. L. Di, and C. Buonavoglia. 2006. Detection of canine distemper virus in dogs by real-time RT-PCR. J. Virol. Methods 136:171-176.
8. Englund L., M. Chriél, H. H. Dietz, and K. O. Hedlund. 2002. Astrovirus epidemiologically linked to pre-weaning diarrhoea in mink. Vet. Microbiol. 85(1):1-11.
9. Evermann, J. F., A. J. McKeirnan, A. W. Smith, D. E. Skilling, and R. L. Ott. 1985. Isolation and identification of caliciviruses from dogs with enteric infections. Am. J. Vet. Res. 46:218-220.
10. Gentsch, J. R., R. I. Glass, P. Woods, V. Gouvea, M. Gorziglia, J. Flores, B. K. Das, and M. K. Bhan. 1992. Identification of group A rotavirus gene 4 types by polymerase chain reaction. J. Clin. Microbiol. 30:1365-1373.
11. Hall, T.A. 1999. BioEdit: a user-friendly biological sequence alignment and analysis program for Windows 95/98/NT. Nucleic. Acids Symp. Ser. 41:95-98.
12. Hoshino Y., J. F. Zimmer, N. S. Moise, and F. W. Scott. 1981. Detection of astroviruses in feces of a cat with diarrhea. Arch. Virol. 70(4):373-376. (Brief report.)
13. Hu, R. L., G. Huang, W. Qiu, Z. H. Zhong, X. Z. Xia, and Z. Yin. 2001. Detection and differentiation of CAV-1 and CAV-2 by polymerase chain reaction. Vet. Res. Commun. 25:77-84.
14. Imada T., S. Yamaguchi, M. Mase, K. Tsukamoto, M. Kubo, and A. Morooka. 2000. Avian nephritis virus (ANV) as a new member of the family Astroviridae and construction of infectious ANV cDNA. J. Virol. 74(18):8487-8493.
15. Jonassen C. M., T. O. Jonassen, and B. Grinde. 1998. A common RNA motif in the 3' end of the genomes of astroviruses, avian infectious bronchitis virus and an equine rhinovirus. J. Gen. Virol. 79(4):715-718.
16. Kjeldsberg E, and A. Hem. 1985. Detection of astroviruses in gut contents of nude and normal mice. Arch. Virol. 84(1-2):135-140. (Brief report.)
17. Koopmans M. P., Bijen M. H., S. S. Monroe, J. Vinjé. 1998. Age-stratified seroprevalence of neutralizing antibodies to astrovirus types 1 to 7 in humans in The Netherlands. Clin. Diagn. Lab. Immunol. 5(1):33-37.
18. Krishna N. K. 2005. Identification of structural domains involved in astrovirus capsid biology. Viral Immunol. 18(1):17-26.
19. Kurtz J.B., and T. W. Lee. 1984. Human astrovirus serotypes. Lancet. 2(8416):1405.
20. Madeley C. R., and B. P. Cosgrove. 1975. 28 nm particles in faeces in infantile gastroenteritis. Lancet. 6;2(7932):451-452. (Letter.)
21. Marshall J. A, D. S. Healey, M. J. Studdert, P. C. Scott, M. L. Kennett, B. K. Ward, and I. D. Gust. 1984. Viruses and virus-like particles in the faeces of dogs with and without diarrhoea. Aust. Vet. J. 61(2):33-38.
22. Martella V., E. Lorusso, N. Decaro, G. Elia, A. Radogna, M. D'Abramo, C. Desario, A. Cavalli, M. Corrente, M. Camero, C. A. Germinario, K. Banyai, B. Di Martino, F. Marsilio, L. E. Carmichael, and C. Buonavoglia. 2008. Detection and molecular characterization of a canine norovirus. Emerg. Infect. Dis. 14(8): 1306-1308.
23. Martella V., N. Decaro, E. Lorusso, A. Radogna, P. Moschidou, F. Amorisco, M. S. Lucente, C. Desario, V. Mari, G. Elia, K. Banyai, L. E. Carmichael, and C. Buonavoglia. 2009. Genetic heterogeneity and recombination in canine noroviruses. J. Virol. 83(21):11391-11396.
24. McNulty M. S., W. L. Curran, J.B. McFerran. 1980. Detection of astroviruses in turkey faeces by direct electron microscopy. Vet. Rec. 106(26):561.
25. Mendez, E., and C. F. Arias. 2007. Astroviruses, p. 981-1000. In D. M. Knipe and P. M. Howley (ed.), Fields virology, 5th ed., vol. 1. Lippincott Willliams & Wilkins, Philadelphia, PA.
26. Méndez-Toss M., P. Romero-Guido, M. E. Munguía, E. Méndez, and C. F. Arias. 2000. Molecular analysis of a serotype 8 human astrovirus genome. J. Gen. Virol. 81(12):2891-2897.
27. Mochizuki, M., A. Kawanishi, H. Sakamoto, S. Tashiro, R. Fujimoto, and M. Ohwaki. 1993. A calicivirus isolated from a dog with fatal diarrhoea. Vet. Rec. 132:221-222.
28. Pollock R.V.H., and L. E. Carmichael. 1982. Maternally derived immunity to canine parvovirus infection: transfer, decline, and interference with vaccination. J. Am. Vet. Med. Assoc. 180:37-42.
29. Rivera R., H. H. Nollens, S. Venn-Watson, F. M. Gulland, and J. F. Wellehan Jr. 2010. Characterization of phylogenetically diverse astroviruses of marine mammals. J. Gen. Virol. 91(1):166-173.
30. San Gabriel, M. C., Y. Tohya, T. Sugimura, T. Shimizu, S. Ishiguro, and M. Mochizuki. 1997. Identification of canine calicivirus capsid protein and its immunoreactivity in western blotting. J. Vet. Med. Sci. 59:97-101.
31. Sanchez-Fauquier A., A. L. Carrascosa, J. L. Carrascosa, A. Otero, R. I. Glass, J. A. Lopez, C. San Martin, and J. A. Melero. 1994. Characterization of a human astrovirus serotype 2 structural protein (VP26) that contains an epitope involved in virus neutralization. Virology. 201(2):312-320.
32. Schaffer, F. L., M. E. Soergel, J. W. Black, D. E. Skilling, A. W. Smith, and W. D. Cubitt. 1985. Characterization of a new calicivirus isolated from feces of a dog. Arch.Virol. 84:181-195.
33. Snodgrass D. R., and E. W. Gray. 1977. Detection and transmission of 30 nm virus particles (astroviruses) in faeces of lambs with diarrhoea. Arch. Virol. 55(4):287-291.
34. Tamura K., J. Dudley, M. Nei and S. Kumar. 2007. MEGA4: Molecular Evolutionary Genetics Analysis (MEGA) software version 4.0. Mol. Biol. Evol. 24:1596-1599
35. Todd D., V. J. Smyth, N. W. Ball, B. M. Donnelly, M. Wylie, N. J. Knowles, and B. M. Adair. 2009. Identification of chicken enterovirus-like viruses, duck hepatitis virus type 2 and duck hepatitis virus type 3 as astroviruses. Avian Pathol. 38(1):21-30.
36. Toffan A., C. M. Jonassen, C. De Battisti, E. Schiavon, T. Kofstad, I. Capua, and G. Cattoli. 2009. Genetic characterization of a new astrovirus detected in dogs suffering from diarrhoea. Vet. Microbiol. 139(1-2):147-152.
37. Tzipori S., J. D. Menzies, and E. W. Gray. 1981. Detection of astrovirus in the faeces of red deer. Vet Rec. 108(13):286.
38. Vennema, H., E. de Bruin, and M. Koopmans. 2002. Rational optimization of generic primers used for Norwalk-like virus detection by reverse transcriptase polymerase chain reaction. J. Clin. Virol. 25:233-235.
39. Vieler E., and W. Herbst. 1995. Electron microscopic demonstration of viruses in feces of dogs with diarrhea. Tierarztl. Prax. 23(1):66-69.
40. Walter J. E., J. Briggs, M. L. Guerrero, D. O. Matson, L. K. Pickering, G. Ruiz-Palacios, T. Berke, and D. K. Mitchell. 2001. Molecular characterization of a novel recombinant strain of human astrovirus associated with gastroenteritis in children. Arch. Virol. 146(12):2357-2367.
41. Wang, Q. H., M. G. Han, S. Cheetham, M. Souza, J. A. Funk, and L. J. Saif. 2005. Porcine noroviruses related to human noroviruses. Emerg. Infect. Dis. 11:1874-1881.
42. Williams F.P. Jr. 1980. Astrovirus-like, coronavirus-like, and parvovirus-like particles detected in the diarrheal stools of beagle pups. Arch. Virol. 66(3):215-226.
43. Woode G. N., and J. C. Bridger. 1978. Isolation of small viruses resembling astroviruses and caliciviruses from acute enteritis of calves. J. Med. Microbiol. 11(4):441-52.

## Claims

1. An isolated canine astrovirus, wherein said astrovirus:
i) encodes a polypeptide SEQ ID NO:1 at the carboxy-terminus of the polymerase complex and a capsid protein SEQ ID NO:2; or a polypeptide having at least 97%, preferably at least 98% sequence identity to SEQ ID NO:1 or SEQ ID NO:2 over the entire sequence;
ii) is able to grow on MDCK cells;
iii) causes gastro-enteritis in dogs.

2. An isolated virus according to claim 1, containing a polynucleotide encoding a polypeptide at the carboxy terminus of the polymerase complex, said polynucleotide being either SEQ ID NO:3 or a polynucleotide hybridizing to full-length SEQ ID NO:3 under stringent conditions.

3. An isolated virus according to claim 1, containing a polynucleotide encoding a capsid protein, said polynucleotide being either SEQ ID NO:4 or a polynucleotide hybridizing to full-length SEQ ID NO:4 under stringent conditions.

4. An isolated virus according to claim 1, containing two open reading frames (ORF) that encode for the polypeptides SEQ ID NO:1 and SEQ ID NO:2, respectively designated ORF1b and ORF2, wherein said ORFs overlap over a 8-nucleotides region between the termination codon of ORF1b and the initiation codon of ORF2.

5. An isolated virus according to claims 1-4, containing a sequence SEQ ID NO:5 spanning the 3' end of ORF1b (SEQ ID NO:3), the full-length ORF2 (SEQ ID NO:4) and the 3' non coding region to the poly-A tail.

6. An isolated canine astrovirus polypeptide selected from SEQ ID NO: 1 and SEQ ID NO:2, or a canine astrovirus polypeptide having at least 95%, preferably at least 97% sequence identity to full-length SEQ ID NO: 1 or SEQ ID NO:2.

7. An isolated canine astrovirus polynucleotide selected from SEQ ID NO:3, SEQ ID NO:4 and SEQ ID NO:5, or an isolated canine astrovirus polynucleotide hybridizing to the full-length sequence SEQ ID NO:3, SEQ ID NO:4 or SEQ ID NO:5 under stringent conditions.

8. An isolated antibody specifically binding to the canine astrovirus according to claims 1-5 or to a polypeptide according to claim 6.

9. A method for determining the presence of an astrovirus of claims 1-5 in a dog, comprising the steps of: (i) providing a dog cell, tissue or body-fluid sample, (ii) contacting that sample with an antibody that binds the canine astrovirus according to claim 8 and (iii) detecting the binding of the antibodies to the sample, whereby detection of a binding is indicative of the presence of the virus in the tested sample.

10. A method for determining the presence of an astrovirus of claims 1-5 in a dog, comprising the steps of: (i) providing a dog body fluid sample, (ii) contacting that sample with a virus polypeptide according to claim 6 adhered to a solid support and determining the formation of an antigen-antibody complex, whereby detection of the antigen-antibody complex is indicative of virus presence in the sample.

11. A method for the detection of an astrovirus according to claims 1-4 in a dog, comprising (i) providing a sample of dog cells, tissues or body fluid and (ii) amplifying a virus specific sequence selected from SEQ ID NO:3, SEQ ID NO:4 and SEQ ID NO:5, or a fragment thereof, by means of PCR techniques, whereby the detection of a sequence amplicon is indicative of virus presence in the tested sample.

12. A vaccine composition containing an immunogenically active component which is selected from: an astrovirus according to claims 1-5, which has been attenuated or inactivated; an isolated astrovirus polypeptide according to claim 6; an isolated canine astrovirus polynucleotide according to claim 7; together with veterinary-compatible carriers and excipients.

13. A vaccine composition according to claim 12, for use in the prevention or treatment of astrovirus-associated gastro-enteritis in dogs, particularly in young animals and pups.


SEQUENCE LISTING

<110>  Martella, Vito

<120>  NOVEL CANINE ASTROVIRUS

<130>  2301EUR

<160>  7

<170>  PatentIn version 3.3

<210>  1
<211>  237
<212>  PRT
<213>  Unknown

<220>
<223>  mamastrovirus

<400>  1

Ile Arg His Ile Lys Lys Leu Arg Trp Ser Leu Val Asn Ala Glu Gln
1               5                   10                  15

Arg Arg Lys Tyr Gln Lys Leu His Asp Trp Tyr Val Glu Asn Leu Val
            20                  25                  30

Asn Arg Thr Val Leu Leu Pro Ser Gly Glu Val Thr Glu Gln His Arg
        35                  40                  45

Gly Asn Pro Ser Gly Gln Phe Ser Thr Thr Met Asp Asn Asn Met Ile
    50                  55                  60

Asn Thr Trp Leu Gln Ala Phe Glu Phe Ala Tyr Phe His Gly Pro Asn
65                  70                  75                  80

Lys Gln Leu Trp Leu Asn Tyr Asp Thr Leu Val Tyr Gly Asp Asp Arg
                85                  90                  95

Leu Ser Thr Thr Pro Leu Ile Pro Asp Asn Tyr Val Glu Arg Val Val
            100                 105                 110

Leu Met Tyr Lys Asp Val Phe Gly Met Trp Val Lys Pro Glu Lys Val
        115                 120                 125

Lys Ile Ser Asn Thr Ile Val Gly Leu Ser Phe Cys Gly Phe Thr Val
    130                 135                 140

Asp Glu Asn Leu Glu Pro Ile Pro Thr Gln Pro Asp Lys Leu Met Ala
145                 150                 155                 160

Ser Leu Leu Lys Pro Ala Ser Lys Leu Pro Asp Leu Glu Ser Leu His
                165                 170                 175

Gly Lys Leu Leu Cys Tyr Gln Leu Leu Ser Ala Phe Leu Pro Glu Glu
            180                 185                 190

His Pro Phe Lys Val Tyr Val Glu Ser Cys Leu Ala Ala Thr Ser Arg
        195                 200                 205

Gln Leu Arg Asp Ser Gly Leu Pro Thr Arg Phe Thr Glu Glu Gln Met
    210                 215                 220

His Arg Ile Trp Arg Gly Gly Pro Lys Asn Cys Asp Gly
225                 230                 235

<210>  2
<211>  774
<212>  PRT
<213>  Unknown

<220>
<223>  mamastrovirus

<220>
<221>  misc_feature
<222>  (495)..(495)
<223>  Xaa can be any naturally occurring amino acid

<400>  2

Met Ala Ser Lys Pro Gly Lys Asp Val Thr Val Glu Val Lys Thr Ser
1               5                   10                  15

Gly Thr Lys Ser Thr Ser Ser Arg Ser Lys Ser Arg Gly Arg Asn Arg
            20                  25                  30

Asn Val Lys Ile Thr Val Asn Ser Gln Pro Lys Thr Asn Arg Arg Arg
        35                  40                  45

Arg Asn Arg Pro Asn Asn Arg Gly Arg Lys Arg Val Glu Ala Val Val
    50                  55                  60

Lys Arg Gln Leu Asp Lys Ala Gly Val Thr Gly Pro Arg Pro Ala Ile
65                  70                  75                  80

Thr Gln Thr Ala Thr Ser Thr Leu Gly Thr Ile Gly Pro Asn Thr Ser
                85                  90                  95

Gly Ala Val Glu Leu Glu Leu Ala Thr Phe Met Asn Pro Cys Leu Val
            100                 105                 110

Lys Glu Ser Thr Ala Ser Asn Ser Phe Gly Pro Ile Gln Ala Ser Ala
        115                 120                 125

Ala Gln Tyr Asn Leu Trp Arg Val Thr Lys Ala Gln Val Arg Leu Thr
    130                 135                 140

Pro Met Val Gly Pro Ser Ala Ile Ser Gly Thr Ala Tyr Arg Val Ser
145                 150                 155                 160

Leu Asn Thr Ala Gly Thr Pro Ser Ser Thr Gly Trp Ser Gly Leu Gly
                165                 170                 175

Ala Arg Lys His Lys Asp Val Lys Val Gly Ser Met Ser Val Phe Thr
            180                 185                 190

Ile Thr Ala Arg Asp Met Ala Gly Pro Arg Glu Gly Trp Phe Val Thr
        195                 200                 205

Asn Thr Asn Glu Ser Gly Gly Glu Ser Val Gly Pro Thr Ile Glu Leu
    210                 215                 220

His Ser Leu Gly Glu Thr Gln Ser Thr Tyr Gln Asn Gln Arg Tyr Thr
225                 230                 235                 240

Gly Pro Val Phe Leu Val Glu Leu His Cys Thr Trp Gln Phe Ser Asn
                245                 250                 255

Tyr Ser Ala Asn Pro Ala Leu Ala Gln Leu Glu Lys Gly Glu Asp Lys
            260                 265                 270

Asp Ala Gln Ile Lys Phe Glu Gly Thr Ala Gly Gln Pro Leu Thr Met
        275                 280                 285

Thr Val Ala Pro His Ser Ala Phe Ala Arg Ala Ile Glu Ser Arg Ser
    290                 295                 300

Thr Val Pro Tyr Ser Gly Ala Gly Arg Ala Ala Gly Glu Ser Thr Ser
305                 310                 315                 320

Asp Thr Ile Trp Gln Ile Ala Ser Thr Ala Val Asp Ala Ala Ser Val
                325                 330                 335

Val Val Pro Pro Pro Phe Asn Trp Leu Ile Lys Gly Gly Trp Trp Phe
            340                 345                 350

Val Lys Lys Leu Ala Gly Arg Ser Arg Thr Gly Glu Ile Gln Leu Gln
        355                 360                 365

Val Phe Ala Ser Tyr Glu Asp Ala Gln Asn Asn Arg Pro Ala Ile Cys
    370                 375                 380

Thr Gly Pro Val Ala Asp Asp Asn Pro Ser Arg Leu His Asn Val Lys
385                 390                 395                 400

Phe Val Gln Met Asn Ala Pro Ser Thr Gly Met Pro Pro Glu Ser Ala
                405                 410                 415

Leu Gly Ala Phe Ser Leu Pro Leu Pro Asp Thr Pro Ala Thr Ile Gly
            420                 425                 430

Asp Ser Phe Arg Leu Ile Thr Gly Val Tyr Gln Gln Phe Gln Gln Asn
        435                 440                 445

Pro Pro Cys Pro Thr Tyr Val Tyr His Ser Thr Asn Lys Met Val Val
    450                 455                 460

Lys Val Gly Thr His Tyr Asp Glu Leu Asn Phe Val Tyr Arg Ala Thr
465                 470                 475                 480

Thr Pro His Ile Trp Leu Ala Gly Gly Pro Gln Thr Trp Gln Xaa Ala
                485                 490                 495

Asp Lys Pro Asp Glu Leu Ser Phe Met Glu Ile Gly Ile Lys Ser Gly
            500                 505                 510

Ser Thr Tyr Leu Lys Arg Gly Ile Val Trp Gly Tyr Ser Gln His Lys
        515                 520                 525

Ile Val Ser Gly Ser Glu Leu Ser Val Leu Thr Phe Tyr Val Gly Lys
    530                 535                 540

Leu Asp Asn Gln Ile Arg Tyr Thr Asn Tyr Lys Lys Val Arg Tyr Leu
545                 550                 555                 560

Phe Thr Gly Thr Gly Ser Ser Leu Asn Leu Ala Pro Asp Ser Thr Thr
                565                 570                 575

Thr Asp Leu Gln Val Leu Glu Leu Asn Pro Gly Pro Trp Phe Val Phe
            580                 585                 590

Ala Ser Leu His Leu Val Asn Ser Ser Gln Gln Gln Ser Tyr Ile Ala
        595                 600                 605

Thr Glu Asn Thr Asn Arg Pro Thr Ile Val Cys Pro Val Asp Ala Gln
    610                 615                 620

Asn Leu Phe Val Cys Thr Asn Asn Asn Val Ala Ser Thr Met Ser Ile
625                 630                 635                 640

Asp Phe Ile Arg Thr Pro Pro Val Phe Thr Gln Gln Val Glu Gln Gln
                645                 650                 655

Gln Val Leu Ala Leu Pro Thr Ile Lys Glu Glu Gln Leu Pro Gln Pro
            660                 665                 670

Ile Glu Asp Gly Phe Leu Pro Asp Ser Glu Asp Glu Asp Phe Ser Asp
        675                 680                 685

Asp Asp Ser Leu Leu Asp Asp Asp Asp Phe Phe Pro Ala Ser Asp Gln
    690                 695                 700

Gln Val Phe Ser Ser Arg Gln Val Leu Phe Arg Ala Met Val Asn Glu
705                 710                 715                 720

Gly Trp Pro Glu Asp Gln Ala Glu Arg Leu Ala Lys Arg Ala Leu Pro
                725                 730                 735

Thr Leu Ser Glu Lys Glu Leu Arg Asp Glu Phe Leu Val Gly Leu Ala
            740                 745                 750

Asp Gly Phe Ser Pro Arg Gln Ala Ala Ala Asn Ala Arg Glu Lys Cys
        755                 760                 765

Ser Arg Gly His Ala Glu
    770

<210>  3
<211>  716
<212>  DNA
<213>  Unknown

<220>
<223>  mamastrovirus

<400>  3
ttattcgaca tatcaagaaa ctccgctgga gtttggttaa tgccgaacaa aggaggaaat      60

accaaaaact tcatgattgg tatgttgaaa atctggtcaa tcgtactgtg ctacttccat      20

ctggcgaggt tacagaacaa catcgtggta atccatctgg ccaattctcc actaccatgg     180

ataacaacat gatcaacaca tggctccagg cttttgaatt tgcctatttc catggaccta     240

acaaacagct ttggctgaac tatgacactt tggtctatgg tgatgacagg ctgtcaacaa     300

ctccacttat tcctgacaac tatgttgaga gagttgtact aatgtacaaa gatgtttttg     360

gaatgtgggt caagcctgag aaagtcaaaa tttcaaacac cattgttggt ctcagttttt     420

gtggctttac tgtagatgag aatcttgaac caatacctac acaacctgat aaattgatgg 480

cctcacttct taaaccagca tctaaacttc cggatcttga atcactccat gggaaactcc 540

tgtgctatca gctcctctcg gctttcctac ctgaggaaca ccctttcaag gtgtatgtcg 600

agagttgtct ggctgccact agcaggcagc ttcgtgattc tggtttacct accagattca 660

cagaagagca aatgcatcgc atatggaggg gaggaccaaa aaattgcgat ggctag 716

<210> 4
<211> 2325
<212> DNA
<213> Unknown

<220>
<223> mamastrovirus

<220>
<221> misc_feature
<222> (1483)..(1483)
<223> n is a, c, g, or t

<400> 4
atggctagca agccaggcaa agatgtcacc gttgaggtta aaacctccgg aacaaaatca 60

acatcctcta ggagcaaatc ccgggggcga aaccggaatg tcaagatcac agtcaattca 120

caacccaaaa caaatcggag gagacgaaac agacctaaca atcgtggtcg caagagagtt 180

gaggctgtcg ttaaacgaca gctcgataaa gctggagtca caggaccaag gccagcgatt 240

acccaaactg ctacatctac tctgggaact attggcccga atacttcggg tgcagtagag 300

ctggaacttg caacattcat gaatccatgc ttggtcaaag agtcaacagc ttccaactct 360

tttggaccca tccaagcatc agctgcacaa tacaatctat ggagggtcac aaaagcccaa 420

gttcgcctga ctccaatggt tggcccatct gcaatctcag gtactgctta tagggtttcc 480

ctaaacaccg cgggaacccc ttcttccaca ggctggtcag gtttgggggc tagaaagcac 540

aaagatgtca aagttggctc catgtctgtt tttaccatca ctgctagaga catggctggc 600

ccacgtgaag gttggtttgt caccaacacc aatgagtcag gtggtgagtc tgttggacct 660

acaattgagt tgcactcact tggtgaaacc cagtcaacct atcaaaatca gaggtacact 720

ggacctgtct tccttgtgga attgcattgc acatggcaat tttcaaacta cagtgcaaat 780

ccagctctgg ctcagctaga gaaaggagag gacaaggacg cacaaatcaa gtttgaaggt 840

acagcaggac aaccacttac catgacagtg gcacctcact cagcttttgc cagagccatt 900

gaatcccgct ccacggttcc ctactcagga gcgggtagag ctgcaggcga atcaacttca 960

gacacaatct ggcaaattgc atctacagct gttgatgctg cttctgtggt tgttccacca 1020

cctttcaatt ggctcatcaa gggtggttgg tggtttgtta agaagctggc agggagatcg 1080

cgtacaggcg agatccaact gcaggtgttt gccagctatg aagatgctca aaataaccgc 1140

ccagccatct gcacagggcc agtagcagac gacaatccct cacgtctgca caatgtgaaa 1200

tttgttcaaa tgaatgcacc atcaacaggt atgccacctg aatctgcatt aggtgctttt 1260

agcctaccac taccagacac tccagcaact attggagaca gttttaggct cataacaggg 1320

gtataccagc aatttcaaca aaaccctccg tgtcctacct atgtttacca tagtaccaac 1380

aagatggtcg ttaaggtagg cacacactat gatgagttga attttgtcta cagagcaaca 1440

acacctcata tttggcttgc tggtggacct caaacttggc agnttgcaga caaaccagat 1500

gagctgtctt ttatggagat tgggatcaaa tcaggttcca cctacctcaa acgtggtatt 1560

gtctggggtt actcacaaca caagatagtg agtgggagcg aactaagcgt acttaccttc 1620

tatgtaggta agctggacaa ccagatacgc tatactaact acaagaaagt tcgctatctt 1680

tttactggga caggttccag tcttaatttg gcaccagatt ccacaaccac tgacctgcag 1740

gtacttgagc ttaaccctgg accctggttt gtgtttgctt ccttgcatct cgtcaactcc 1800

tcacaacagc agagctacat tgcaactgag aacacaaaca gacctacaat tgtttgccct 1860

gttgatgcgc aaaacttgtt tgtttgtacc aacaacaatg ttgccagcac aatgtcaatt 1920

gatttcatca gaacaccacc tgttttcaca caacaagtgg aacaacaaca agttcttgct 1980

cttcctacca tcaaagagga acaattgcca cagcccattg aagatggttt tctcccagat 2040

tctgaagatg aagatttttc tgatgatgat tctcttcttg atgatgatga ttttttccct 2100

gcttctgatc agcaggtttt ttccagtcgc caggtccttt tcagagcaat ggtcaatgag 2160

ggttggccag aggaccaggc tgagcgcctt gccaagcgcg ctctccctac actaagtgag 2220

aaagaactta gggatgaatt tttagtcgga cttgctgacg gcttctcgcc acgtcaagca 2280

gccgcaaatg ctcgtgaaaa gtgttcccga ggccacgccg agtag 2325

<210> 5
<211> 3143
<212> DNA
<213> Unknown

<220>
<223> mamastrovirus


<220>
<221> misc_feature
<222> (2191)..(2191)
<223> n is a, c, g, or t

<400> 5
ttattcgaca tatcaagaaa ctccgctgga gtttggttaa tgccgaacaa aggaggaaat 60

accaaaaact tcatgattgg tatgttgaaa atctggtcaa tcgtactgtg ctacttccat 120
ctggcgaggt tacagaacaa catcgtggta atccatctgg ccaattctcc actaccatgg 180
ataacaacat gatcaacaca tggctccagg cttttgaatt tgcctatttc catggaccta 240
acaaacagct ttggctgaac tatgacactt tggtctatgg tgatgacagg ctgtcaacaa 300
ctccacttat tcctgacaac tatgttgaga gagttgtact aatgtacaaa gatgtttttg 360
gaatgtgggt caagcctgag aaagtcaaaa tttcaaacac cattgttggt ctcagttttt 420
gtggctttac tgtagatgag aatcttgaac caatacctac acaacctgat aaattgatgg 480
cctcacttct taaaccagca tctaaacttc cggatcttga atcactccat gggaaactcc 540
tgtgctatca gctcctctcg gctttcctac ctgaggaaca ccctttcaag gtgtatgtcg 600
agagttgtct ggctgccact agcaggcagc ttcgtgattc tggtttacct accagattca 660
cagaagagca aatgcatcgc atatggaggg gaggaccaaa aaattgcgat ggctagcaag 720
ccaggcaaag atgtcaccgt tgaggttaaa acctccggaa caaaatcaac atcctctagg 780
agcaaatccc gggggcgaaa ccggaatgtc aagatcacag tcaattcaca acccaaaaca 840
aatcggagga gacgaaacag acctaacaat cgtggtcgca agagagttga ggctgtcgtt 900
aaacgacagc tcgataaagc tggagtcaca ggaccaaggc cagcgattac ccaaactgct 960
acatctactc tgggaactat tggcccgaat acttcgggtg cagtagagct ggaacttgca 1020
acattcatga atccatgctt ggtcaaagag tcaacagctt ccaactcttt tggacccatc 1080
caagcatcag ctgcacaata caatctatgg agggtcacaa aagcccaagt tcgcctgact 1140
ccaatggttg gcccatctgc aatctcaggt actgcttata gggtttccct aaacaccgcg 1200
ggaacccctt cttccacagg ctggtcaggt ttgggggcta gaaagcacaa agatgtcaaa 1260
gttggctcca tgtctgtttt taccatcact gctagagaca tggctggccc acgtgaaggt 1320
tggtttgtca ccaacaccaa tgagtcaggt ggtgagtctg ttggacctac aattgagttg 1380
cactcacttg gtgaaaccca gtcaacctat caaaatcaga ggtacactgg acctgtcttc 1440
cttgtggaat tgcattgcac atggcaattt tcaaactaca gtgcaaatcc agctctggct 1500
cagctagaga aaggagagga caaggacgca caaatcaagt ttgaaggtac agcaggacaa 1560
ccacttacca tgacagtggc acctcactca gcttttgcca gagccattga atcccgctcc 1620
acggttccct actcaggagc gggtagagct gcaggcgaat caacttcaga cacaatctgg 1680
caaattgcat ctacagctgt tgatgctgct tctgtggttg ttccaccacc tttcaattgg 1740
ctcatcaagg gtggttggtg gtttgttaag aagctggcag ggagatcgcg tacaggcgag 1800
atccaactgc aggtgtttgc cagctatgaa gatgctcaaa ataaccgccc agccatctgc 1860
acagggccag tagcagacga caatccctca cgtctgcaca atgtgaaatt tgttcaaatg 1920

aatgcaccat caacaggtat gccacctgaa tctgcattag gtgcttttag cctaccacta 1980

ccagacactc cagcaactat tggagacagt tttaggctca taacaggggt ataccagcaa 2040

tttcaacaaa accctccgtg tcctacctat gtttaccata gtaccaacaa gatggtcgtt 2100

aaggtaggca cacactatga tgagttgaat tttgtctaca gagcaacaac acctcatatt 2160

tggcttgctg gtggacctca aacttggcag nttgcagaca aaccagatga gctgtctttt 2220

atggagattg ggatcaaatc aggttccacc tacctcaaac gtggtattgt ctggggttac 2280

tcacaacaca agatagtgag tgggagcgaa ctaagcgtac ttaccttcta tgtaggtaag 2340

ctggacaacc agatacgcta tactaactac aagaaagttc gctatctttt tactgggaca 2400

ggttccagtc ttaatttggc accagattcc acaaccactg acctgcaggt acttgagctt 2460

aaccctggac cctggtttgt gtttgcttcc ttgcatctcg tcaactcctc acaacagcag 2520

agctacattg caactgagaa cacaaacaga cctacaattg tttgccctgt tgatgcgcaa 2580

aacttgtttg tttgtaccaa caacaatgtt gccagcacaa tgtcaattga tttcatcaga 2640

acaccacctg ttttcacaca acaagtggaa caacaacaag ttcttgctct tcctaccatc 2700

aaagaggaac aattgccaca gcccattgaa gatggttttc tcccagattc tgaagatgaa 2760

gatttttctg atgatgattc tcttcttgat gatgatgatt ttttccctgc ttctgatcag 2820

caggtttttt ccagtcgcca ggtccttttc agagcaatgg tcaatgaggg ttggccagag 2880

gaccaggctg agcgccttgc caagcgcgct ctccctacac taagtgagaa agaacttagg 2940

gatgaatttt tagtcggact tgctgacggc ttctcgccac gtcaagcagc cgcaaatgct 3000

cgtgaaaagt gttcccgagg ccacgccgag taggatcgag ggtacaggtt cactttcact 3060

cttttctttt ctgtctttaa caatcactta tatagttaag ttggattagt ttaggcattg 3120

aaaaaaaaaa aaaaaaaaaa aaa 3143

<210> 6
<211> 23
<212> DNA
<213> Unknown

<220>
<223> synthetic oligonucelotide

<400> 6
gtactatacc rtctgattta att 23

<210> 7
<211> 22
<212> DNA
<213> Unknown

<220>

<223>  synthetic oligonucelotide

<400>  7
agaccaargt gtcatagttc ag                                                22